# EUROPEAN PATENT APPLICATION

(11) **EP 4 397 386 A1**
(43) Date of publication of application: **10.07.2024**
(21) Application number: 22864466.2
(22) Date of filing: 29.08.2022
(51) Int. Cl.: B01D 15/08, B01D 15/38, B01J 19/00, B01J 20/285, B81B 1/00, B81C 1/00, C07B 57/00, G01N 30/88, G01N 35/10, G01N 37/00

(54) **MICROFLUIDIC DEVICE AND METHOD FOR MANUFACTURING SAME**

(30) Priority: 30.08.2021 JP 2021140379
(71) Applicant: Daicel Corporation, Osaka 530-0011 (JP); The University of Tokyo, Bunkyo-ku, Tokyo 113-8654 (JP)
(72) Inventor: TSUNODA, Makoto, Tokyo 113-8654 (JP); FUKUDA, Daisuke, Tokyo 108-8230 (JP); ONISHI, Takafumi, Tokyo 108-8230 (JP); NOZOKIDO, Takashi, Myoko-shi, Niigata 944-8550 (JP)
(74) Representative: Grünecker Patent- und Rechtsanwälte PartG mbB
(86) International application number: PCT/JP2022/032330
(87) International publication number: WO 2023/032883

(57) **Abstract**

A microfluid device includes: a separation channel having a tunnel shape; an obstacle having a columnar shape and provided in the separation channel; and a ligand supported on a surface of the obstacle, and the ligand is an optically active polymer.

## Description

### TECHNICAL FIELD

The present disclosure relates to a microfluid device and a method for producing the same.

### BACKGROUND ART

In the related art, as a method for separating a specific component from a sample in which a plurality of components is mixed, a method using a device having a separation function such as liquid chromatography including a separation column as a stationary phase is known. As a separation column used for general liquid chromatography, a particle-packed column in which particles such as particulate silica gel are packed in a cylinder, or a monolithic column in which silica gel or the like having a three-dimensional network structure is provided in a cylinder has been used.

In recent years, liquid chromatography including, as a stationary phase, a column having a pillar array structure in which a plurality of columnar obstacles (pillars) is arranged in a tunnel-shaped separation channel has been developed and studied. In a case of the particle-packed column or the monolithic column, unfortunately, it is difficult to precisely control a size and arrangement of particles and a size and arrangement of meshes forming the three-dimensional network structure. In contrast, in a case of the pillar array column, pillars can be produced and arranged using a technique such as semiconductor microfabrication, and thus, there is an advantage that a structure in accordance with a design drawing can be precisely prepared to achieve high separation efficiency.

As a technique related to the pillar array structure, Patent Document 1 discloses a technique related to a mixer capable of realizing high mixing characteristics while maintaining a separation state of separated components by providing a separation channel in which a shape and a size of pillars and a distance between pillars are designed within specific numerical ranges. In addition, Non-Patent Document 1 discloses that in a pillar array column having a turn structure, high separation efficiency can be achieved by forming a channel into a low-dispersion shape. Non-Patent Document 2 discloses that in a pillar array column having a turn structure, high separation efficiency can be achieved by controlling arrangement of pillars in a channel.

### PRIOR ART DOCUMENT

### PATENT DOCUMENT

[Patent Document 1] JP 2018-122295 A

### NON-PATENT DOCUMENT

[Non-Patent Document 1] Chiaki Aoyama, et al., Analytical Chemistry, 82, 1420-1426 (2010)
[Non-Patent Document 2] Muneki Isokawa, et al., Analytical Chemistry, 88, 6485-6491 (2016)

### SUMMARY OF THE INVENTION

### PROBLEMS TO BE SOLVED BY THE INVENTION

For imparting separation ability to a device, in Patent Document 1, a plurality of pillars are formed in a separation channel, and in Non-Patent Documents 1 and 2, after a plurality of pillars is formed in a separation channel, a treatment for chemically bonding an octadecylsilyl group to surfaces of the pillars is further performed. However, no method for separating optical isomers is disclosed.

Optical isomers are used in a wide range of technical fields, and particularly in fields of pharmaceuticals, agrochemicals, and the like, there are many optically active compounds in substances to be used, and research and development on optical isomers have been actively conducted. For this reason, it is a great advantage that optical isomers can be separated from a sample, in which a plurality of components is mixed, in a separation column used in a device such as liquid chromatography. That is, there is room for improvement in the above-described known devices, particularly in a microfluid device, which cannot separate optical isomers.

Accordingly, the present disclosure is directed to providing a microfluid device capable of separating optical isomers.

### MEANS FOR SOLVING THE PROBLEMS

As a result of intensive studies, the present inventors have found that the above-described issue can be solved by causing a surface of a pillar provided in a separation channel to support an optically active polymer as a ligand, and have reached the present disclosure.

[1] A microfluid device including:
   a separation channel having a tunnel shape;
   an obstacle having a columnar shape and provided in the separation channel; and
   a ligand supported on a surface of the obstacle, the ligand being an optically active polymer.
[2] The microfluid device according to [1], wherein the optically active polymer is a polysaccharide or a derivative thereof.
[3] The microfluid device according to [1] or [2], wherein the separation channel has a turn structure, and the turn structure is a structure formed in a tapered shape such that a shape formed by an inner wall surface in a plan view expands toward an outer peripheral side.
[4] The microfluid device according to [3], wherein, in the turn structure, a length of an outer periphery and a length of an inner periphery are identical in a plan view.
[5] The microfluid device according to [1] or [2], wherein the separation channel has a turn structure, and the turn structure has a gradient in which a density of the number of pillars decreases from an inner peripheral side to the outer peripheral side.
[6] The microfluid device according to any one of [1] to [5], further including a sample channel for introducing a sample.
[7] The microfluid device according to [6], wherein the separation channel and the sample channel are provided on a same substrate.
[8] The microfluid device according to [7], wherein a shape of the substrate is a chip shape.
[9] A method for producing a microfluid device, the method including:
   preparing a substrate including a separation channel having a tunnel shape and an obstacle having a columnar shape and provided in the separation channel; and
   causing a solution containing a ligand to pass through the separation channel to cause the ligand to be supported on a surface of the obstacle,
   the ligand being an optically active polymer.
[10] The method for producing a microfluid device according to [9], wherein a shape of the substrate is a chip shape.
[11] The method for producing a microfluid device according to [9] or [10], wherein the substrate further includes a sample channel for introducing a sample.

### EFFECT OF THE INVENTION

According to the present disclosure, it is possible to provide a microfluid device capable of separating optical isomers.

### BRIEF DESCRIPTION OF DRAWINGS

FIG. 1 is a schematic perspective view illustrating an embodiment of a pillar array column.
FIG. 2 is a cross-sectional view illustrating an embodiment of a turn structure.
FIG. 3 is a diagram for explaining a method for producing a ligand-free substrate.
FIG. 4 is a view illustrating an embodiment of a microfluid device.
FIG. 5 is a view illustrating an embodiment of the microfluid device.
FIG. 6 is a view illustrating an embodiment of the microfluid device.
FIG. 7 is a diagram showing a chromatograph according to a result of separation evaluation in an Example.
FIG. 8 is a diagram showing a chromatograph according to a result of separation evaluation in an Example.
FIG. 9 is a diagram showing a chromatograph according to a result of separation evaluation in an Example.

### MODE FOR CARRYING OUT THE INVENTION

Hereinafter, although embodiments of the present disclosure will be described in detail, each of configurations, combinations thereof, and the like in the embodiments are an example, and various additions, omissions, substitutions, and other changes may be made as appropriate without departing from the gist of the present disclosure. The present disclosure is not limited by the embodiments and is limited only by the claims.

In the present description, a numerical range expressed by "to" implies a range including the numerical values described before and after "to" as the lower limit value and the upper limit value. Specifically, "A to B" implies a value that is A or more and B or less.

In addition, in the present description, "a plurality of" implies "two or more".

Note that the schematic diagrams in the drawings illustrate various members appropriately made large or small for the purpose of explanation, and do not illustrate actual sizes or ratios of the embodiments of the present disclosure.

### <Configuration and Characteristics of Microfluid Device>

A microfluid device according to an embodiment of the present disclosure (also simply referred to as a "microfluid device") includes a separation channel having a tunnel shape, an obstacle having a columnar shape (also referred to as a "pillar") and provided in the separation channel, and a ligand supported on a surface of the obstacle, and the ligand is an optically active polymer.

In the microfluid device according to the present embodiment, the optically active polymer is supported on the surface of the pillar, and thus an optical isomer can be separated from a fluid caused to pass through the separation channel having a tunnel shape.

In the present disclosure, "an element including the separation channel having a tunnel shape, the obstacle having a columnar shape and provided in the separation channel, and the ligand supported on the surface of the obstacle" provided in the microfluid device is also referred to as a pillar array column, and the pillar array column will be described below.

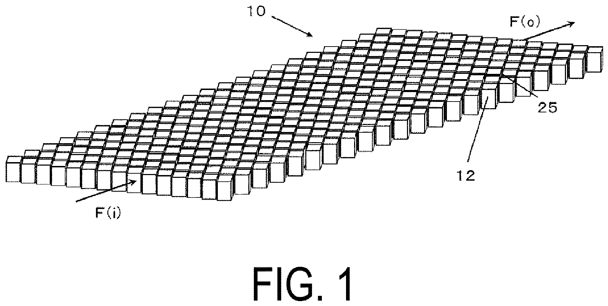

FIG. 1 illustrates an example of the pillar array column provided in the microfluid device according to the present embodiment. A pillar array column 10 illustrated in FIG. 1 includes a tunnel-shaped separation channel formed of a separation channel wall (not illustrated) and columnar obstacles (pillars) 12 provided in the separation channel. An arrow F(i) in FIG. 1 indicates an inflow direction of a fluid into a channel, and an arrow F(o) indicates an outflow direction of the fluid from the separation channel.

In the present disclosure, unless otherwise specified, a numerical condition in which a plurality of objects may be present indicates an average value thereof. Specifically, for example, a height of a pillar is a numerical value calculated by averaging numerical values of heights of a plurality of pillars.

Hereinafter, each component of the pillar array column 10 will be described in detail.

### [Tunnel-Shaped Separation Channel]

The pillar array column 10 includes a tunnel-shaped separation channel. A form of the tunnel-shaped separation channel is not particularly limited, and a shape of a cross-section of the separation channel (the cross-sectional shape of the separation channel) orthogonal to the flow direction (the direction of the arrow F(i) in FIG. 1) may be, for example, a polygonal shape such as a rectangular shape, a circular shape, a semicircular shape, or an elliptical shape, and is preferably a rectangular shape.

A cross-sectional area of the separation channel orthogonal to the flow direction is not particularly limited, but is usually 0.01 µm² or more, preferably 1 µm² or more, more preferably 10 µm² or more, even more preferably 100 µm² or more, particularly preferably 1000 µm² or more, and particularly preferably 5000 µm² or more, from the viewpoint of production stability. The cross-sectional area is usually 1000000 µm² or less, preferably 500000 µm² or less, more preferably 100000 µm² or less, even more preferably 50000 µm² or less, and particularly preferably 30000 µm² or less, from the viewpoint of utilization as the microfluid device.

A height of the separation channel (particularly, a height of a rectangular shape in a height direction of the pillar in a case where a shape of the cross-section of the separation channel orthogonal to the flow direction is the rectangular shape) is not particularly limited. However, the height is usually 0.1 µm or more, preferably 1 µm or more, more preferably 5 µm or more, and even more preferable 10 µm or more, from the viewpoint of production stability, and is usually 1000 µm or less, preferably 500 µm or less, more preferably 100 µm or less, and still more preferably 50 µm or less, from the viewpoint of utilization as the microfluid device. A length of a width of the separation channel (particularly, a length of a width of a rectangular shape in a case where a shape of a cross-section of the separation channel orthogonal to the flow direction is the rectangular shape) is not particularly limited. However, the length of the width is usually 0.1 µm or more, preferably 1 µm or more, more preferably 5 µm or more, even more preferably 10 µm or more, and particularly preferably 100 µm or more, from the viewpoint of production stability, and is usually 10000 µm or less, preferably 5000 µm or less, more preferably 1000 µm or less, and still more preferably 500 µm or less, from the viewpoint of utilization as the microfluid device.

A length of the separation channel in the flow direction (separation channel length) is not particularly limited. However, the length is usually 1000 mm or less, preferably 750 mm or less, more preferably 500 mm or less, even more preferably 300 mm or less, and particularly preferably 200 mm or less, from the viewpoint of easily reducing the size of the device, shortening the time required for separation, and suppressing the production cost. The lower limit does not particularly need not to be set and may be 1 mm or more, 10 mm or more, or 50 mm or more.

The separation channel may include only a linear structure or may have a curved structure, and the curved structure preferably has a turn structure from the viewpoint of achieving reduction in size of the pillar array column 10. The turn structure means a structure in which a direction of the separation channel is changed, for example, the direction of the separation channel is changed by 10° or more, 45° or more, 90° or more, 110° or more, 130° or more, 150° or more, 160° or more, or 170° or more, and 200° or less, or 190° or less, preferably at least 180°.

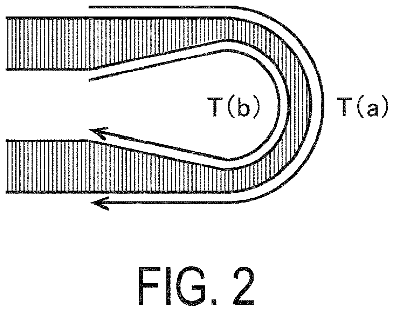

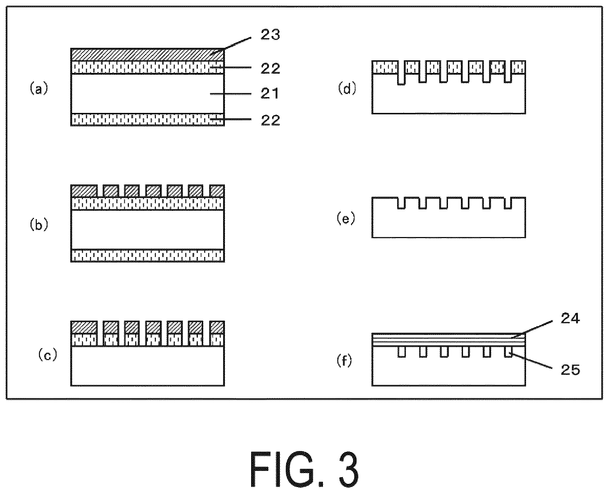

A shape of the turn structure is not particularly limited. However, from the viewpoint of suppressing diffusion of the fluid in a turn portion and channel resistance of the separation channel, the turn structure is preferably formed in a tapered shape such that a shape formed by an inner wall surface in a plan view expands toward an outer peripheral side as disclosed in documents such as Chiaki Aoyama, et al., Analytical Chemistry, 82, 1420 to 1426 (2010) (Non-Patent Document 1), and as the tapered structure, a length of an outer periphery and a length of an inner periphery in a plan view are preferably identical. However, the two lengths being identical includes a case where they are substantially identical, and includes not only a case where the two lengths completely match but also a case where there is an error (for example, a difference of less than ±1% of the length of the outer periphery) with which they can be recognized as being substantially identical (for example, within a range in which the effect of the present disclosure can be achieved). Note that conditions disclosed in known documents such as Non-Patent Document 1 can be applied to the tapered structure as appropriate; however, the tapered structure is not limited thereto and can be designed as appropriate in accordance with usage. The structure formed in a tapered shape such that the shape formed by an inner peripheral side wall surface in a plan view expands toward the outer peripheral side means a structure formed in the following manner: when a length of an inner periphery in a structure produced by bending a channel length having a constant width in a U-shape (for example, a structure as shown in FIG. 3) is defined as U, a length of an inner periphery of the U-shape is made larger than U (while maintaining a shape of an outer peripheral wall surface) (more specifically, a shape formed by the inner peripheral wall surface expands and a side of the inner peripheral wall surface is tapered). FIG. 2 illustrates an example of the tapered structure (no pillar in the separation channel is illustrated). Directions of arrows T(a) and T(b) in FIG. 2 indicate feeding directions of the fluid in the separation channel, a length of T(a) is the length of the outer periphery, and a length of T(b) is the length of the inner periphery.

In addition, from the viewpoint of suppressing diffusion of the fluid and channel resistance in the turn portion, preferably, the channel has a turn structure and the turn structure has a gradient in which a density of the number of pillars decreases from the inner peripheral side to the outer peripheral side, as disclosed in documents such as Muneki Isokawa, et al., Analytical Chemistry, 88, 6485 to 6491

(2016) (Non-Patent Document 2). Note that conditions disclosed in known documents such as Non-Patent Document 2 can be appropriately applied to the structure having the gradient; however, the structure is not limited thereto and can be appropriately designed in accordance with usage.

The number of turn structures (the number of turns) of the pillar array column is not particularly limited and can be designed as appropriate in accordance with usage. The number of turn structures may be 1, 2 or more, 4 or more, 6 or more, 8 or more, or 10 or more, and 1000 or less, or 100 or less. The number of turn structures is preferably an even number from the viewpoint of suppressing diffusion of the fluid (the diffusion is canceled by a flow from the inside to the outside and a flow from the outside to the inside).

A shape of a member forming the separation channel is not particularly limited and may be any shape. For example, the member may have a shape produced by a method for producing a ligand-free substrate described below, or may have the same shape as the separation channel, that is, may have a shape formed of a wall having a certain thickness to surround the separation channel. As a method of forming the separation channel, for example, in a case where the separation channel is formed of a wall having a constant thickness to surround the separation channel, a method of forming the separation channel by combining sheet members serving as separation channel walls can be employed. In a case where the separation channel has any shape, a method of providing a member having the shape with a through-hole serving as the separation channel can be employed.

A material of the member forming the separation channel, that is, the material of the channel wall is not particularly limited, but is particularly preferably silicon from the viewpoint of ease of semiconductor processing.

### [Columnar Obstacle (Pillar)]

The pillar array column 10 includes columnar obstacles (pillars) 12. A shape of each of the pillars 12 is not particularly limited, and a shape of a bottom surface of the pillar 12 may be a polygonal shape such as a triangular shape, a quadrangular shape (particularly a rectangular shape), a pentagonal shape, or a hexagonal shape, or a cylindrical shape. However, from the viewpoint of ease of semiconductor processing, the shape of the bottom surface of the pillar 12 is preferably a quadrangular shape, and particularly preferably a rectangular shape. The shape of the bottom surface of each of the pillars 12 in the pillar array column 10 in FIG. 1 is a rectangular shape.

The arrangement of the pillars is not particularly limited; however, from the viewpoint of preventing peak broadening and column pressure increase, the pillars are preferably uniformly arranged.

The member forming the separation channel and the pillars may be produced separately and then joined to each other, or may be produced as an integrally molded body.

An area of the bottom surface of each of the pillars 12 is not particularly limited. However, the area is usually 0.01 µm² or more, preferably 0.1 µm² or more, more preferably 1 µm² or more, and even more preferably 5 µm² or more, from the viewpoint of production stability, and is usually 1000000 µm² or less, preferably 100000 µm² or less, more preferably 10000 µm² or less, even more preferably 1000 µm² or less, more preferably 100 µm² or less, and further particularly preferably 50 µm² or less, from the viewpoint of utilization as the microfluid device.

The maximum width of the bottom surface of each of the pillars 12 (a length of the possible maximum line segment in the bottom surface of the pillar) is not particularly limited. However, the maximum width is usually 0.1 µm or more, preferably 0.5 µm or more, more preferably 1 µm or more, and still more preferably 2 µm or more, from the viewpoint of production stability, and is usually 1000 µm or less, preferably 100 µm or less, more preferably 50 µm or less, and still more preferably 10 µm or less, from the viewpoint of utilization as the microfluid device.

In a case where the shape of the bottom surface of each of the pillars 12 is a quadrangular shape, a length of each side is not particularly limited. However, the length is usually 0.1 µm or more, preferably 0.5 µm or more, more preferably 1 µm or more, and still more preferably 2 µm or more, from the viewpoint of production stability, and is usually 1000 µm or less, preferably 100 µm or less, more preferably 50 µm or less, and still more preferably 10 µm or less, from the viewpoint of utilization as the microfluid device.

A height of each of the pillars 12 is not particularly limited. The height is not particularly limited, but is usually 0.1 µm or more, preferably 1 µm or more, more preferably 5 µm or more, and still more preferably 10 µm or more, from the viewpoint of production stability, and is usually 1000 µm or less, preferably 500 µm or less, more preferably 100 µm or less, and still more preferably 50 µm or less, from the viewpoint of utilization as the microfluid device.

The smallest distance between pillars (the smallest distance between side surfaces of pillars) is not particularly limited. However, the smallest distance is usually 0.1 µm or more, preferably 0.5 µm or more, more preferably 1 µm or more, and still more preferably 1.5 µm or more, from the viewpoint of production stability, and is usually 1000 µm or less, preferably 100 µm or less, more preferably 50 µm or less, and still more preferably 10 µm or less, from the viewpoint of utilization as the microfluid device.

A pitch distance between pillars (the smallest distance between central axes of pillars) is not particularly limited. However, the pitch distance is usually 0.1 µm or more, preferably 0.5 µm or more, more preferably 1 µm or more, and still more preferably 3 µm or more, from the viewpoint of production stability, and is usually 1000 µm or less, preferably 100 µm or less, more preferably 50 µm or less, and still more preferably 10 µm or less, from the viewpoint of utilization as the microfluid device.

The number of pillars in the separation channel is not particularly limited and can be appropriately designed in accordance with an amount of a sample to be separated and a size of an apparatus.

The pillars each support an optically active polymer as a ligand on the surface thereof. This makes it possible to separate optical isomers from a fluid caused to pass through the tunnel-shaped separation channel.

A type of the optically active polymer is not particularly limited. Examples thereof include polysaccharides or derivatives thereof, poly(meth)acrylic acid amides, polyamino acids, and polyamides. From the viewpoint of high separation performance, polysaccharides or derivatives thereof are preferable.

A type of the polysaccharides or derivatives thereof is not particularly limited. Examples of the polysaccharides include β-1,4-glucan (cellulose), α-1,4-glucan (amylose, amylopectin), α-1,6-glucan (dextran), β-1,6-glucan (pustulan), β-1,3-glucan (curdlan, sizofiran), α-1,3-glucan, β-1,2-glucan (Crown Gall polysaccharide), β-1,4-galactan, β-1,4-mannan, α-1,6-mannan, β-1,2-fructan (inulin), β-2, 6-fructan (levan), β-1,4-xylan, β-1,3-xylan, β-1,4-chitosan, β-1,4-N-acetylchitosan (chitin), pullulan, agarose, alginic acid, α-cyclodextrin, β-cyclodextrin, γ-cyclodextrin, nigeran, and amylose.

Among them, from the viewpoint of easily producing a polysaccharide of high purity, cellulose, amylose, β-1,4-chitosan, chitin, β-1,4-mannan, β-1,4-xylan, inulin, curdlan, pullulan, α-cyclodextrin, β-cyclodextrin, γ-cyclodextrin, or nigeran is preferable, and cellulose, amylose, pullulan, or nigeran is more preferable.

One of the optically active polymers can be used alone, or two or more thereof can be used in combination.

A number average degree of polymerization (an average number of pyranose or furanose rings contained in one molecule) of the polysaccharide is preferably 5 or greater, and more preferably 10 or greater. Although there is no particular upper limit, the number average degree of polymerization is preferably 1000 or less from the viewpoint of ease of handling, more preferably 5 or greater and 1000 or less, still more preferably 10 or greater and 1000 or less, and particularly preferably 10 or greater and 500 or less.

With respect to these polysaccharides, for example, an ester derivative or a carbamate derivative produced by chemically modifying cellulose or amylose can be used as the ligand.

Such a polysaccharide derivative is known to have a high optical resolution ability as a chiral stationary phase.

Specific examples of the ester derivative and the carbamate derivative that can be used as the ligand of the present disclosure include a cellulose derivative in which a hydroxyl group of cellulose is modified with a substituent produced by substituting a part of hydrogen of an aromatic ring of phenyl carbamate with halogen (fluorine or chlorine) as described in JP H04-42371 A, and a cellulose derivative and an amylose derivative in which a hydroxyl group of cellulose or amylose is modified with a substituent produced by substituting a part of hydrogen of an aromatic ring of phenyl carbamate with fluorine, an alkyl group, or an alkoxy group as described in JP 2005-315668 A.

Among the polysaccharides or derivatives thereof described above, the above-mentioned polysaccharide derivatives are preferably used from the viewpoint of separation performance of optical isomers to be separated and from the viewpoint of ease of being supported on the pillars.

The polysaccharide derivatives are not limited to those described above, and any desired type can be used as appropriate.

A method for causing the pillars to support the polysaccharide or the derivative thereof is not particularly limited, and the polysaccharide or the derivative thereof may be supported by physical adsorption or may be supported by chemical bonding. Examples of the physical adsorption method include a method in which a pillar is coated with an optically active polymer, and examples of the chemical bonding method include chemical bonding between the pillar and the polysaccharide derivative, chemical bonding using a third component, and a method in which chemical bonding is generated by reaction caused by light irradiation, radiation irradiation such as γ-ray, or electromagnetic wave irradiation such as microwave, or radical reaction using a radical initiator.

A supported amount of the optically active polymer in the separation channel (an amount of the optically active polymer supported on the pillars, in particular, an amount of the optically active polymer supported on the pillars and the separation channel wall) is not particularly limited as long as the effect of the present disclosure can be achieved. However, from the viewpoint of stable separation of optical isomers, the supported amount is usually 0.001 µmol/m² or more, preferably 0.02 µmol/m² or more, and more preferably 0.1 µmol/m² or more, and usually 2.0 µmol/m² or less, preferably 1.0 µmol/m² or less, and more preferably 0.9 µmol/m² or less, in terms of an average amount per unit area of the separation channel in a plan view.

A compound other than the above-described optically active polymer may be supported on the pillars, and examples thereof include optically inactive polyesters such as polyethylene terephthalate, polybutylene terephthalate, polyethylene-polybutylene terephthalate, polytrimethylene terephthalate, polyethylene naphthalate, polybutylene naphthalate, polylactic acid, polyglycolic acid, poly ε-caprolactone, and poly(oxycarbonyloxy-1,4-phenylene-2,2-isopropylidene-1,4-phenylene) {polycarbonate of bisphenol A); proteins such as protein A, protein G, protein L, albumin, and functional variants thereof; nucleic acids such as DNA, RNA, oligonucleotides, and modified oligonucleotides; and other low-molecular compounds or oligomers.

### <Method for Producing Microfluid Device>

A method for producing a microfluid device according to another embodiment of the present disclosure (hereinafter, also simply referred to as "method for producing a microfluid device") includes a substrate preparation process of preparing a substrate including a tunnel-shaped separation channel and a columnar obstacle provided in the separation channel, and a supporting process of causing a solution containing a ligand to pass through the separation channel to allow the ligand to be supported on a surface of the obstacle, and the ligand is an optically active polymer. The terms and conditions in the above description of the microfluid device can be similarly applied to the present embodiment related to the production method unless otherwise specified.

In the present disclosure, a substrate including a pillar array column in which no ligand is supported on pillars is also referred to as a ligand-free substrate.

### [Substrate Preparation Process]

The method for producing a microfluid device according to the present embodiment includes a substrate preparation process of preparing a substrate (ligand-free substrate) including a tunnel-shaped separation channel and a columnar obstacle provided in the separation channel.

The method for producing the substrate is not particularly limited, and the substrate can be produced by a known method or a combination of known methods. However, from the viewpoint that the substrate can be produced in accordance with a design drawing, a method including a process using a semiconductor processing technique, specifically, a process of patterning the substrate using a semiconductor processing technique is preferable. A shape of the substrate is not particularly limited, but is preferably a chip shape from the viewpoint of ease of handling and ease of production. In the present disclosure, a chip-shaped substrate (sometimes simply referred to as a "chip") means a single plate-shaped member.

A form of the substrate is not particularly limited as long as the pillar array column can be provided thereon, and a commercially available product can be used. In a case of using the chip-shaped substrate, for example, the chip-shaped substrate may be cut out from a bulk material made of silicon or the like, may be formed by curing a composition containing a material such as silicon, or may be a commercially available product as it is.

A specific example of the method for producing a ligand-free substrate will be described with reference to FIG. 3.

A photosensitive resin 23 is applied onto one surface of a silicon substrate 21 having oxide films 22 on both front and back surfaces (FIG. 3(a)), and this surface is subjected to lithographic exposure through a mask in accordance with a design drawing of the separation channel and development (photolithography) to form a resin pattern on the substrate (FIG. 3(b)). Thereafter, the substrate is immersed in acid to perform wet ething of a portion of the silicon oxide film which is not covered with the resin (FIG. 3(c)). Further, the residual resin is removed, and deep-reactive ion etching (deep-RIE) is performed using the oxide film as a resist to etch the separation channel (FIG. 3(d)). Thereafter, the residual oxide film is removed by wet ething (FIG. 3(e), at the time, the waste generated in the etching can be removed), then, the surface of the silicon substrate is reoxidized by Oz plasma (for a surface modification reaction to be performed later), and finally the substrate is bonded to a glass substrate 24 by anodic bonding to form a separation channel 25 (FIG. 3(f)), thereby completing production of a ligand-free substrate.

A material of the substrate is not limited to silicon, and materials including the materials of the channel wall described above can be used.

The operations of lithographic exposure and development, pattern formation, etching, anodic bonding, and the like described above can be performed by known methods.

### [Supporting Process]

The method for producing a microfluid device according to the present embodiment includes a supporting process of causing a solution containing a ligand to pass through the separation channel to allow the ligand to be supported on the surface of the obstacle (pillar).

The method for causing the solution of the ligand to pass through the channel is not particularly limited, and can be performed by a known method, and for example, the solution containing the ligand can be caused to pass through from F(i) to F(o) in FIG. 1. A flow rate at the time of passing through is not particularly limited, and may be, for example, 0.1 cm³/s or more and 10.0 cm³/s or less, or 1.0 cm³/s or more and 5.0 cm³/s or less.

After causing the solution to pass through, the pillar array column is subjected to natural drying, drying under reduced pressure, heating treatment, or the like to dry the solution of the ligand present (applied) on the pillars in the separation channel, thereby allowing the ligand to be supported (coated) on the pillar. In a case where the heating treatment is performed, a heating temperature is not particularly limited and may be set depending on the solution to be used. For example, the heating temperature may be 10°C or higher and 100°C or lower, or 30°C or higher and 80°C or lower.

The solution containing a ligand is not particularly limited as long as it contains a ligand. The ligand is not particularly limited as long as it is an optically active polymer, and the above-described optically active polymers can be used.

A content of the ligand (in particular, the optically active polymer) in the solution to be caused to pass through to attach the optically active polymer to the pillars is not particularly limited as long as the effect of the present disclosure can be achieved. However, from the viewpoint of stable separation of optical isomers, the content of the ligand is usually 0.01 mmol/L or more, preferably 0.1 mmol/L or more, and more preferably 0.5 mmol/L or more, and is usually 2.0 mmol/L or less, preferably 1.5 mmol/L or less, and more preferably 1.0 mmol/L or less.

A type of a solvent in the solution containing a ligand is not particularly limited as long as it can dissolve the ligand, and may be, for example, acetone, tetrahydrofuran, dimethylformamide, dimethylacetamide, or the like. Among them, acetone is preferable from the viewpoint of achieving good separation performance.

One of the solvents can be used alone or two or more thereof can be used in combination.

The solution containing a ligand may contain a component (an additional component) other than the ligand and the solvent.

### [Additional process]

The method for producing a microfluid device according to the present embodiment may include a process (additional process) other than the substrate preparation process and the supporting process, and may include, for example, a synthesis process of synthesizing a ligand (an optically active polymer), a solution preparation process of dissolving a ligand in a solvent to produce a solution containing the ligand, or the like.

In addition, in a case where a mixer or the like that may be provided in a microfluid device described below is provided, a process of forming the mixer, a process of forming an inlet for introducing a derivatization reagent, and a process of forming a derivatization reagent channel can also be provided.

The method for synthesizing a ligand in the synthesis process is not particularly limited, and the ligand can be synthesized by a known method or a combination of known methods depending on the type of the ligand.

In the solution preparation process, a method for performing dissolution is not particularly limited, and may be performed by a known method. For example, a ligand may be added to a solvent to perform dissolution while being stirred and further appropriately heating the mixture.

A method for forming each member in a process of forming a mixer, a process of forming an inlet for introducing a derivatization reagent, and a process of forming a derivatization reagent channel can be the same as the method for forming a separation channel described above.

The microfluid device including the pillar array column according to the present disclosure can be used as a device of liquid chromatography or the like for separating a specific component from a sample in which a plurality of components is mixed.

Note that in the present disclosure, the microfluid device means a device having a channel or a structure on the order of µm, and the size of the entire device does not need to be on the order of µm.

In a microfluid device according to another embodiment of the present disclosure, the microfluid device according to the above-described embodiment (preferably, the substrate of the microfluid device) further includes a sample channel for introducing a sample. The form in which the microfluid device includes the sample channel is not particularly limited. In an aspect, the sample channel is formed in the microfluid device itself, for example, formed by performing patterning for forming the sample channel in the microfluid device having the pillar array column formed therein by the same method as the above-described method in which the pillar array column is provided on the substrate using patterning. In another aspect, the sample channel is formed by connecting another member including the sample channel to the microfluid device. Among these aspects, from the viewpoint of ease of production, production stability, and miniaturization, in a preferable aspect, the sample channel is formed in the microfluid device itself, and in a particularly preferable aspect, the separation channel and the sample channel are provided on the same substrate. Hereinafter, an aspect in which the separation channel and the sample channel are provided on the same substrate will be specifically described.

In the microfluid device according to the present embodiment described above, the sample channel and the separation channel are formed on the same substrate, and thus, as compared to a device in which the sample channel and the separation channel exist as separate members and production is performed by bonding them, it is easy to reduce the size of the device and shorten the time required for separation, and it is also possible to suppress the production cost.

The method for forming the sample channel in the microfluid device is not particularly limited. However, from the viewpoint that the microfluid device can be produced in accordance with a design drawing, it is preferable to apply a method using the semiconductor processing technique for forming the separation channel in the substrate described above, specifically, a method including a process of using the semiconductor processing technique to pattern a substrate.

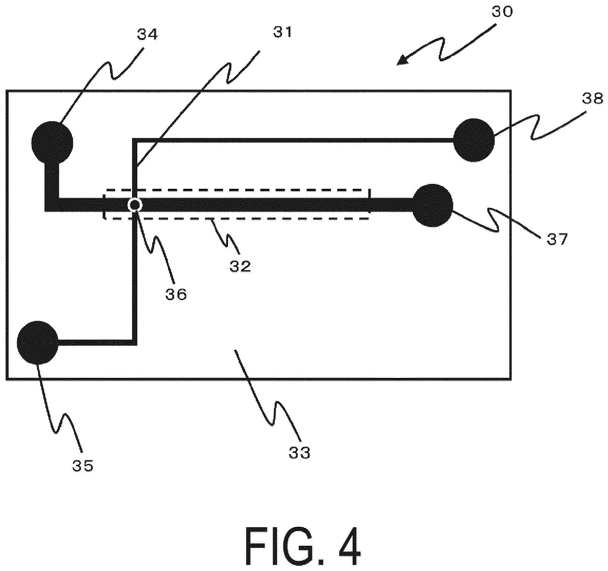

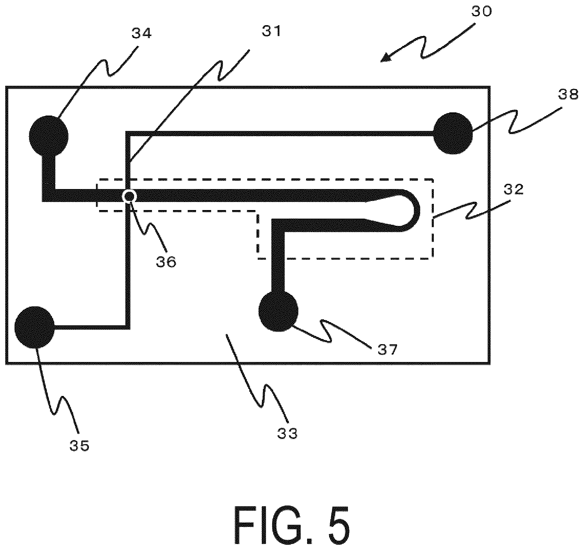

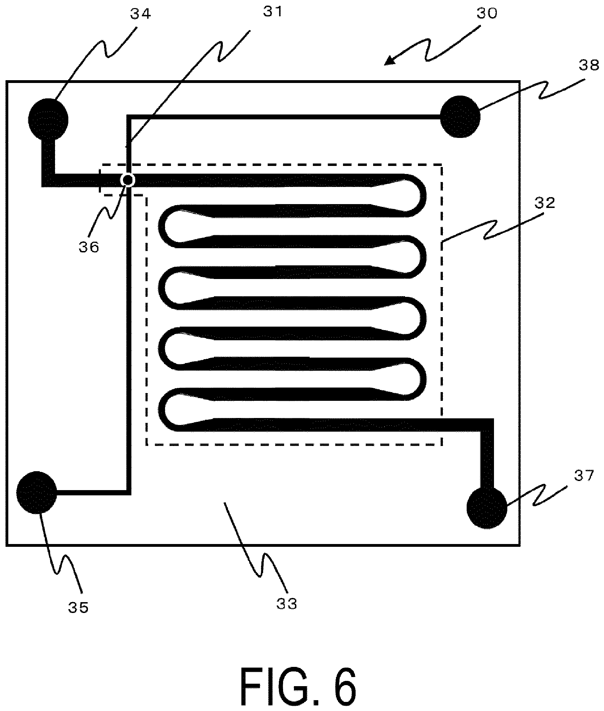

An example of the microfluid device according to the present embodiment is illustrated in FIG. 4. In a microfluid device 30 illustrated in FIG. 4, a sample channel 31 for introducing a sample and a separation channel 32 for separating components in the sample by flowing them together with a mobile phase are formed on the same substrate (herein also referred to as a " chip " (chip-shaped substrate)) 33. In FIG. 4, for reasons of expressing all members in black, a portion of the separation channel 32 (a portion of the channel in which pillars are present) is expressed by being surrounded by a dotted line (although a sample injection portion 36 is present in the range of the dotted line, a portion in which the pillars are present is expressed by the dotted line, and the pillars can be arranged in the sample injection portion 36). The dotted line has the same meaning in FIGS. 5 and 6 to be described below. In addition, in the present disclosure, a region where the pillars are present in a portion through which the mobile phase and the sample are caused to pass is referred to as a separation channel.

Note that a form of the mobile phase and the sample channel before and after the separation channel 32 in FIG. 4 is not particularly limited, and may be any form as long as the mobile phase and the sample can pass therethrough. For example, the form may be a tunnel-shaped cavity formed by removing the pillars from the separation channel. In addition, the mobile phase channel through which only the mobile phase flows (the channel through which the mobile phase flows before the mobile phase and the sample are mixed) is not particularly limited as long as the mobile phase can pass through the channel.

In FIG. 4, the microfluid device includes a mobile phase inlet 34, from which the mobile phase such as water, an organic solvent, or a buffer is introduced into the device, and a sample inlet 35, from which a sample is introduced into the device. The sample introduced from the sample inlet 35 is mixed with the mobile phase introduced from the mobile phase inlet 34 in the sample injection portion 36, and introduced into the separation channel 32 together with the mobile phase. Components contained in the sample introduced together with the mobile phase are separated in the flow direction in the separation channel 32. Note that the sample and the mobile phase introduced into the separation channel 32 are discharged from an outlet 37. In this case, the sample and the mobile phase flow into the separation channel along F(i) illustrated in FIG. 1. Meanwhile, a sample introduced into a sample processing apparatus from the sample inlet 35 and not mixed with the mobile phase in the sample injection portion 36 is discharged from a sample outlet 38. Note that portions from the sample inlet 35 to the sample outlet 38 are connected by a sample channel 31 which is a groove formed in the chip 33. Note that the separation channel 32, the mobile phase inlet 34, the sample inlet 35, the outlet 37, and the like included in the microfluid device 30 are formed as grooves on a substrate made of silicon or the like, a mixer or the like is also formed as necessary, and upper portions (opening portions) of the grooves are covered with glass as illustrated in FIG. 4.

The method for feeding a liquid to the mobile phase inlet 34 is not particularly limited. For example, the liquid can be fed by using a micro liquid chromatography pump while the liquid can be fed by pressure-feeding to the sample inlet 35.

A form of the substrate is not particularly limited. For example, a shape of a surface of the substrate on which the separation channel is provided may be a polygonal shape such as a rectangular shape, a circular shape, a semicircular shape, an elliptical shape, or the like, and is preferably a rectangular shape. The shape of the substrate may be a polygonal plate such as a rectangular plate, a circular plate, a semicircular plate, an elliptical plate, or the like, and is preferably a rectangular plate.

Hereinafter, parameters related to the form of the substrate will be described, but these parameters can also be treated as parameters of the form of the microfluid device within an applicable range.

An area of a surface of the substrate on which the pillar array column is provided is not particularly limited, but is usually 100 mm² or more and preferably 400 mm² or more from the viewpoint of ease of handling, and is usually 10000 mm² or less and preferably 5000 mm² or less from the viewpoint of cost reduction.

A thickness of the substrate is not particularly limited, but is usually 100 µm or more and preferably 150 µm or more from the viewpoint of ease of handling, and is usually 1000 µm or less and preferably 500 µm or less from the viewpoint of cost reduction.

In addition, in a case where the shape of the surface of the substrate on which the pillar array column is provided is a rectangular shape, a length of each side is not particularly limited, but is usually 10 mm or more and preferably 20 mm or more from the viewpoint of ease of handling, and is usually 100 mm or less and preferably 70 mm or less from the viewpoint of cost reduction.

With the above-described configuration, it is possible to cause a mobile phase and a sample to flow into the separation channel in the microfluid device, thereby separating optical isomers.

The microfluid device according to the present embodiment has a form as illustrated in FIG. 4 in a case where the separation channel is configured only in a linear shape, but can have a form as illustrated in FIG. 5 in a case where the separation channel has the above-described turn structure. The number of turn structures is not limited, and in a case where a plurality of turn structures is used, a form as illustrated in FIG. 6 can be employed.

Note that the turn structures of the separation channels illustrated in FIGS. 5 and 6 are each a structure formed in a tapered shape such that the shape formed by the inner wall surface in a plan view expands toward the outer peripheral side. However, the shape of the turn structure is not limited to this shape and any turn structure may be employed.

The microfluid device according to the present embodiment may include a member other than the members illustrated in FIG. 4 on the same substrate 33, and may include, for example, a mixer for derivatizing a separated substance between the separation channel 32 and the outlet 37. In a case where a mixer is provided, an inlet for introducing a derivatization reagent and a derivatization reagent channel can also be provided.

### EXAMPLES

Hereinafter, the present disclosure will be specifically described with reference to Examples. The present disclosure is not to be interpreted as being limited to Examples described below.

### < Example 1>

### [Production of Ligand-Free Substrate]

As the microfluid device, in the configuration illustrated in FIG. 6 (provided that the number of turn structures of the separation channel was 12), a groove having a depth of 60 µm was formed as the sample channel 31 on a square microchip having a side of 20 mm and a thickness of 500 µm, and the other channels through which the mobile phase and the sample flow were formed as grooves having a depth of 30 µm. The separation channel 32 had a width of 400 µm (a width of the thinnest portion in a curved portion of the turn structure was 110 µm).

The separation channel 32 had a width of 400 µm (a length in the direction orthogonal to the flow direction) and a length of 110 mm in the flow direction, and the pillars in the separation channel 32 were aligned in 3 µm in side length and 2 µm in inter-plane spacing.

In the microfluid device described above, each structure was formed on a silicon substrate by a photolithography method and deep reactive ion etching known in the related art, and then the substrate was bonded to glass by an anodic bonding method known in the related art to produce a ligand-free substrate.

### [Supporting of Ligand]

500 mg of a cellulose derivative (Cellulose tris(3,5-dimethylphenylcarbamate)) was dissolved in 1 L of acetone. Subsequently, a syringe pump (YSP-101 "standard type", available from YMC Co., Ltd.) was used to cause this solution to flow to the ligand-free substrate at room temperature (25°C) and a flow rate of 2 µL/min for 60 minutes. Thereafter, the chip was vacuum-dried at a temperature of 60°C for 6 hours (degree of reduced pressure < 10 mmHg) (rectangular vacuum constant-temperature drier DP300, available from Yamato Scientific Co., Ltd.) to perform supporting of the ligand, thereby producing a microfluid device.

### <Separation Evaluation>

In the microfluid device described above, water/acetonitrile (available from FUJIFII,M Wako Pure Chemical Corporation) of 40/60 (v/v) from the mobile inlet 34 and Flurbiprofen (available from FUJIFILM Wako Pure Chemical Corporation) fluorescence-derivatized with 4-bromomethyl-7-methoxycoumarin (Br-Mmc) represented by the following formula (1) from the sample inlet 35 were fed at a flow rate of 0.5 µL/min, a sample concentration of 100 µM, and a detection position near Outlet (near 110 mm of the column length).

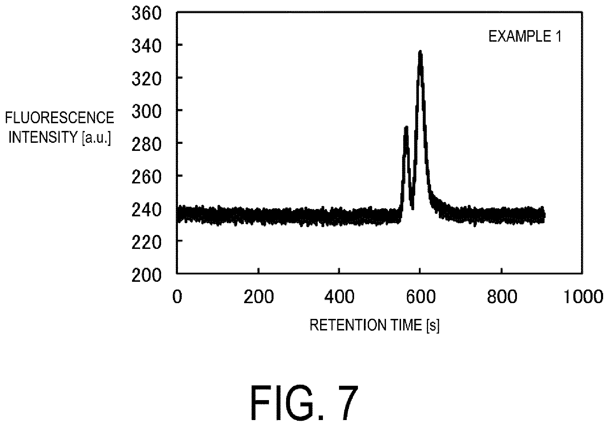

During the separation of the sample, a moving image was taken with a fixed field of view using a fluorescence microscope. From this moving image, a chromatogram was constructed by plotting a temporal change of the fluorescence intensity of a portion on the channel using Andor SOLIS (ver. 4.28.30001.0: Andor Technologies, South Windsor, CT, USA). A back pressure during liquid feeding was recorded in accordance with a pressure value displayed on a monitor of the micropump. The evaluation results are shown in FIG. 7.

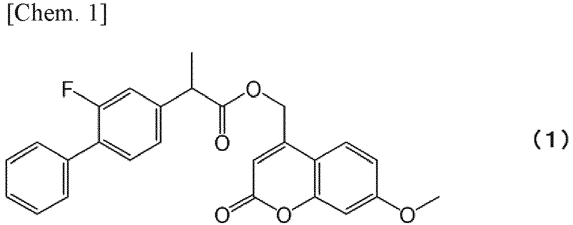

### < Example 2>

### [Production of Ligand-Free Substrate]

A ligand-free substrate was produced in the same manner as in Example 1.

### [Supporting of Ligand]

Supporting of the ligand was performed in the same manner as in Example 1 except that the amount of the cellulose derivative (cellulose tris(3,5-dimethylphenylcarbamate)) was changed from 500 mg to 10 mg to produce a microfluid device.

### <Separation Evaluation>

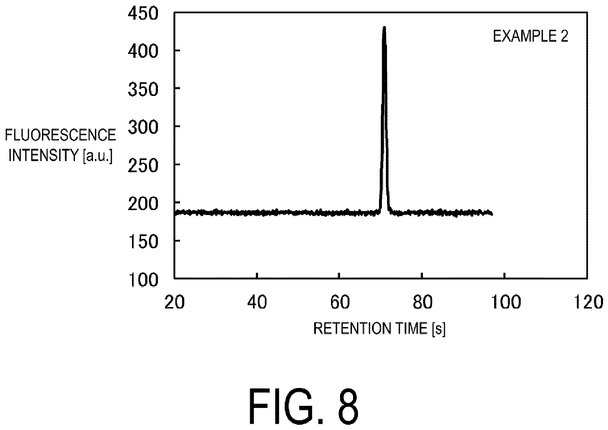

Separation evaluation was performed in the same manner as in Example 1. The evaluation results are shown in FIG. 8.

### < Example 3>

### [Production of Ligand-Free Substrate]

A ligand-free substrate was produced in the same manner as in Example 1.

### [Supporting of Ligand]

Supporting of the ligand was performed in the same manner as in Example 1 except that the amount of the cellulose derivative (cellulose tris(3,5-dimethylphenylcarbamate)) was changed from 500 mg to 100 mg to produce a microfluid device.

### <Separation Evaluation>

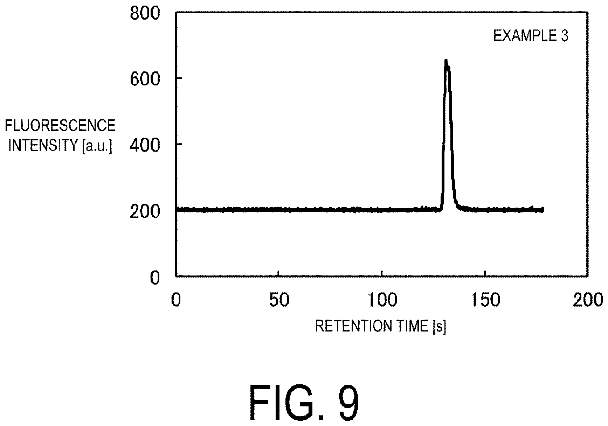

Separation evaluation was performed in the same manner as in Example 1 except that the ratio of water/acetonitrile (available from FUJIFILM Wako Pure Chemical Corporation) from the mobile phase inlet 34 was changed from 40/60 (v/v) to 50/50 (v/v). The evaluation results are shown in FIG. 9.

### REFERENCE SIGNS LIST

10 Pillar array column
12 Pillar
21 Silicon substrate
22 Oxide film
23 Photosensitive resin
24 Glass substrate
25 Separation channel
30 Microfluid device
31 Sample channel
32 Separation channel
33 Chip
34 Mobile phase inlet
35 Sample inlet
36 Sample injection portion
37 Outlet
38 Sample outlet

## Claims

1. A microfluid device comprising:
a separation channel having a tunnel shape;
an obstacle having a columnar shape and provided in the separation channel; and
a ligand supported on a surface of the obstacle, the ligand being an optically active polymer.

2. The microfluid device according to claim 1, wherein the optically active polymer is a polysaccharide or a derivative thereof.

3. The microfluid device according to claim 1 or 2, wherein the separation channel has a turn structure, and the turn structure is a structure formed in a tapered shape such that a shape formed by an inner wall surface in a plan view expands toward an outer peripheral side.

4. The microfluid device according to claim 3, wherein in the turn structure, a length of an outer periphery and a length of an inner periphery in a plan view are identical.

5. The microfluid device according to claim 1 or 2, wherein the separation channel has a turn structure, and the turn structure has a gradient in which a density of the number of pillars decreases from an inner peripheral side to an outer peripheral side.

6. The microfluid device according to any one of claims 1 to 5, further comprising a sample channel for introducing a sample.

7. The microfluid device according to claim 6, wherein the separation channel and the sample channel are provided on a same substrate.

8. The microfluid device according to claim 7, wherein a shape of the substrate is a chip shape.

9. A method for producing a microfluid device, the method comprising:
preparing a substrate including a separation channel having a tunnel shape and an obstacle having a columnar shape and provided in the separation channel; and
causing a solution containing a ligand to pass through the separation channel to allow a surface of the obstacle to support the ligand,
the ligand being an optically active polymer.

10. The method for producing a microfluid device according to claim 9, wherein a shape of the substrate is a chip shape.

11. The method for producing a microfluid device according to claim 9 or 10, wherein the substrate further includes a sample channel for introducing a sample.
